# EUROPÄISCHE PATENTSCHRIFT

(11) **EP 1 374 790 B1**
(45) Veröffentlichungstag und Bekanntmachung des Hinweises auf die Patenterteilung: **22.12.2010**
(21) Anmeldenummer: 02013579.4
(22) Anmeldetag: 20.06.2002
(51) Int. Cl.: A61B 19/00, A61M 1/00

(54) **Verfahren und Vorrichtung zur Vorbereitung einer Drainage**
Method and device to prepare a drainage
Méthode et appareil pour préparer un drainage

(43) Veröffentlichungstag der Anmeldung: 02.01.2004
(73) Patentinhaber: BrainLAB AG, 85622 Feldkirchen (DE)
(72) Erfinder: Pedain, Christoph, 81667 München (DE); Hartlep, Andreas, Dr., 80803 München (DE)
(74) Vertreter: Schwabe - Sandmair - Marx

(56) Entgegenhaltungen:
- US-A- 5 154 723
- US-A- 5 460 490
- US-A- 5 919 135
- US-B1- 6 282 437
- US-B1- 6 331 181

## Beschreibung

Die vorliegende Erfindung bezieht sich auf ein Verfahren und eine Vorrichtung zur Vorbereitung einer Drainage, wie zum Beispiel einer interstitiellen Drainage, insbesondere auf die möglichst optimale Positionierung mindestens eines Katheters zur Durchführung einer Drainage.

Ist zum Beispiel im Kopf einer Person, insbesondere im interstitiellen Raum zum Beispiel aufgrund eines Unfalls oder einer Erkrankung eine übermäßige Menge an Flüssigkeit vorhanden, so kann diese zu Überdruck und damit einem Druck auf das Gehirn führen. Die Verringerung eines solchen Überdruckes sollte so schnell wie möglich durchgeführt werden, um Gesundheitsschäden zu vermeiden. Gewöhnlich wird hierzu ein Katheter an einer Stelle gesetzt, um diese Flüssigkeit aus dem Kopf abfließen zu lassen. Bei einer solchen Drainage kann zum Beispiel ein Katheter an einer für günstig gehaltenen Stelle angebracht und die interstitielle Flüssigkeit durch diesen Katheter aus dem Kopf entfernt werden.

Bisher ist es nicht möglich sicherzustellen, dass möglichst die gesamte unerwünschte Flüssigkeit entfernt werden kann. Aufgrund von personenspezifisch unterschiedlichen Körper- und Gewebestrukturen können Katheter leicht unbeabsichtigt an weniger geeigneten Stellen positioniert werden, so dass die Drainage nur unvollständig durchgeführt werden kann und eventuell ein weiterer Katheter gesetzt werden muss, wodurch weitere Eingriffe erforderlich werden.

Es ist bekannt Medikamente zum Beispiel durch eine Infusion oder eine Injektion beispielsweise über einen Katheter in einen Körper einzubringen. Insbesondere im Falle einer Infusion kann es dabei zum Aufbau eines Überdruckes in dem Körper, zum Beispiel aufgrund einer in das Interstitium eingebrachten Substanz, kommen. Die Verteilung einer durch Infusion eingebrachten Substanz hängt zum Beispiel von den anatomischen Gegebenheiten, dem Infusionsdruck, den verwendeten Kathetern oder der Beschaffenheit der eingebrachten Substanz selbst ab und kann nach dem Einbringen der Substanz außer durch eine Veränderung des Einbringdruckes im Wesentlichen nicht mehr beeinflusst werden.

Aus der US 5,919,135 ist ein System und ein Verfahren zur Behandlung von Krebs bekannt, wobei eine Echtzeitsteuerung verwendet wird, um die Infusion von Medikamenten durchzuführen. Ein Katheter dient zur Entnahme von Blutproben.

Die US 6,282,437 B1 beschreibt das Tracking eines Instruments während eines chirurgischen Eingriffes.

Aus der US 5,154,723 ist eine Vorrichtung bekannt, mit welcher ein Hämatom aus einem Gehirn entfernt werden kann. Dabei soll eine "Drainage-Tube" positioniert werden, wobei durch CT im mikroskopischen Bereich überprüft wird, ob die Positionierung der "Tube" hinreichend genau ist.

Die US 4,930,525 offenbart ein Führungsverfahren zum genauen Platzieren eines Katheters in Verbindung mit einem CT-Scanner, wobei mittels des CT-Scanners ein Querschnittbild des Zielgebietes innerhalb des Körpers erstellt wird und ein Insertionsweg auf dem gescannten Querschnittbild ausgewählt wird, um das Zielgebiet zu erreichen, wobei die laterale Distanz zwischen der vertikalen Achse des gescannten Bildes und der Insertionsstelle bestimmt wird.

Es ist eine Aufgabe der vorliegenden Erfindung Verfahren und eine Vorrichtung zur Vorbereitung einer Drainage, insbesondere zum optimalen Positionieren mindestens eines Katheters bevorzugt für eine interstitielle Drainage vorzuschlagen, mit welchen eine Drainage möglichst gut mit möglichst wenigen Kathetern durchgeführt werden kann. Allgemein soll bevorzugt mit einem Minimum an Eingriffen oder Kathetereinstichen ein Maximum an Flüssigkeit drainiert werden, so dass durch minimal invasive Eingriffe Nebeneffekte soweit wie möglich verringert werden können und mögliche Behandlungsrisiken des Patienten weitgehend ausgeschlossen werden können.

Diese Aufgabe wird durch das Verfahren und die Vorrichtung gemäß den unabhängigen Ansprüchen gelöst. Vorteilhafte Ausführungsformen ergeben sich aus den Unteransprüchen.

Gemäß einem ersten Aspekt bezieht sich die Erfindung auf ein Verfahren zur Anordnung mindestens eines Katheters an einem Körper, insbesondere an einem Kopf, wobei körperspezifisch die individuelle anatomische Struktur und insbesondere die Gewebestruktur erfasst wird. Weiterhin wird körperspezifisch die Lage der interstitiellen Flüssigkeit, insbesondere die Position relativ zu umliegenden Gewebe- oder Körperstrukturen, die Menge und Verteilung erfasst. Allgemein ist es vorteilhaft alle den Abfluss der Flüssigkeit beeinflussende Parameter zu ermitteln, wie zum Beispiel auch die Art oder Zusammensetzung der Flüssigkeit, die Druckverteilung oder andere Parameter. Zur Erfassung der oben erwähnten Daten und Informationen können beispielsweise bildgebende Verfahren, wie Kernspinresonanz(MRI)-, Computertomographie(CT)-, Ultraschall-, Röntgen-, SPECT-, PET- oder andere geeignete Verfahren verwendet werden. Vorteilhaft können auch weitere Untersuchungen oder Messungen durchgeführt werden, zum Beispiel um einen Druck oder die Druckverteilung der interstitiellen Flüssigkeit oder die Zusammensetzung dieser Flüssigkeit zu ermitteln. Erfindungsgemäß werden die so gewonnenen für einen Körper oder Kopf spezifischen Informationen ausgewertet und basierend darauf wird eine möglichst optimale Positionierung eines oder mehrerer Katheter ermittelt. Dabei können zum Beispiel in einem Körper-Koordinatensystem oder einem systemspezifischen Koordinatensystem eine oder mehrere geeignete Positionen für Katheter vorgegeben werden, an welchen ein oder mehrere Katheter gleichzeitig oder nacheinander gesetzt werden können, um eine möglichst optimale Drainage durchzuführen, wobei so viel überschüssige interstitielle Flüssigkeit wie möglich mit einem Minimum an Einstichen drainiert werden sollte. Beispielsweise wird eine Position für einen Katheter ermittelt, bei welcher ein Katheter einen im Körper oder Kopf vorherrschenden Überdruck so schnell wie möglich abbauen kann, wobei optional weitere Positionen ermittelt werden, um das möglichst vollständige Drainieren der interstitiellen Flüssigkeit zu gewährleisten.

Gemäß einem weiteren Aspekt bezieht sich die Erfindung auf ein Verfahren zur Simulation des Abflusses von Flüssigkeit durch einen Katheter, welche in einem Körper, insbesondere im Interstitium vorhanden ist, wobei wie oben beschrieben individuell körperspezifisch anatomische Daten zur Struktur des Körpers und/oder Gewebestruktur und körperspezifisch die Lage, Menge, Verteilung und/oder Art der interstitiellen Flüssigkeit ermittelt wird und weiterhin davon ausgegangen wird, dass ein oder mehrere Katheter an einer oder mehreren vorgegebenen Stellen gleichzeitig oder nacheinander positioniert werden, so dass aus diesen Informationen der Verlauf der Drainage, insbesondere der Abfluss von Flüssigkeit aus dem Körper simuliert werden kann, um so geeignete Positionen zum Anbringen eines oder mehrerer Katheter zu finden oder bezüglich ihrer Effektivität zu überprüfen.

Vorteilhaft können mit einer erfindungsgemäßen Simulation auch andere Drainageparameter ermittelt, optimiert oder überprüft werden, wie zum Beispiel ein Flussrate, ein am Katheter anliegender Unterdruck, Kathetergeometrien oder ähnliches.

Bevorzugt wird das Simulationsverfahren unter der Annahme durchgeführt, dass die Katheter wie oben beschrieben an dem Körper positioniert sind.

Vorteilhaft werden der oder die Katheter unter Verwendung bekannter Navigationsverfahren an die gewünschte Position an dem Körper gebracht. Dabei können an einem Katheter aktive oder passive Marker, wie zum Beispiel reflektierende Oberflächen angebracht sein.

Bevorzugt können den Abfluss der interstitiellen Flüssigkeit beeinflussende Parameter, wie zum Beispiel das Flussverhalten einer bestimmten Flüssigkeit in einem spezifischen Gewebetyp ermittelt und zur Bestimmung der optimalen Position eines Katheters oder zur Simulation verwendet werden. Solche körperspezifischen Parameter können beispielsweise in einer Datenbank gespeichert sein und vor der Durchführung der erfindungsgemäßen Verfahren durch Untersuchungen ermittelt worden sein.

Ebenso können Parameter, welche die Eigenschaft der zu drainierenden Flüssigkeit beschreiben, in einer Datenbank vorhanden sein und zum Beispiel zur Planung der Anordnung eines Katheters oder zur Simulation des Abflusses der Flüssigkeit verwendet werden. Beispielsweise kann die Viskosität, die Wechselwirkung einer bestimmten Flüssigkeit mit einem bestimmten Gewebe, das Flussverhalten in einem bestimmten Gewebetyp, oder andere Informationen in einer Datenbank gespeichert werden, um basierend darauf die erfindungsgemäßen Verfahren durchführen zu können.

Bevorzugt wird zur Durchführung des erfindungsgemäßen Verfahren eines Datenbank verwendet, welche Informationen und Parameter zu einem oder mehreren zur Verfügung stehenden unterschiedlichen Kathetertypen enthält. Beispielhaft können in der Datenbank Informationen über die Geometrie, insbesondere den Durchmesser des Katheters, das Material, die Oberfläche und Eigenschaften davon in Wechselwirkung mit Gewebe oder zu drainierenden Flüssigkeiten in einer Datenbank vorhanden sein, wobei die Auswahl eines oder mehrerer Katheter automatisch und/oder durch einen Benutzer erfolgen kann.

Vorteilhaft können vom erfindungsgemäßen Verfahren zur Anordnung mindestens eines Katheters an einem Körper Informationen zu einer möglicherweise vorteilhaften Anpassung, Veränderung oder Bearbeitung von zu verwendeten Kathetern ermittelt und ausgegeben werden. Beispielsweise kann eine optimale Katheterlänge ermittelt werden, so dass ein für das erfindungsgemäße Verfahren zu verwendender standardisierter Katheter auf eine gewünschte Länge abgeschnitten oder auf eine andere Art modifiziert werden kann.

Bevorzugt können weitere die Drainage beeinflussende Parameter, wie zum Beispiel ein an einen Katheter anzulegender Unterdruck oder eine Saugstärke ermittelt werden, mit welchen ein möglichst gutes Drainageergebnis erhalten werden kann. Hierdurch kann die Abflussrate der interstitiellen Flüssigkeit beeinflusst und geregelt werden, welche gewöhnlich im Bereich einiger ml/h liegt.

Vorteilhaft kann ein Verifikationsverfahren durchgeführt werden, d. h. es kann beispielsweise intraoperativ eine weitere Datenerfassung zum Beispiel durch ein bildgebendes Verfahren oder eine andere Messung durchgeführt werden, um die sich durch die Drainage verändernden körperspezifischen Strukturen und/oder die Position, Verteilung und Menge der interstitiellen Flüssigkeit nach einer teilweise oder vollständig durchgeführten Drainage zu ermitteln. Diese Informationen können einerseits zur Verifikation einer durchgeführten Drainage verwendet werden und können andererseits auch zu einer möglicherweise erforderlichen Korrektur oder Neuplatzierung eines oder mehrerer Katheter, zum Austausch von Kathetern, zum Verändern der Flussrate oder zur Beeinflussung anderer für die Drainage relevanter Parameter verwendet werden, um zum Beispiel für den weiteren Verlauf der Drainage zu berücksichtigen, dass sich durch einen bereits reduzierten Druck oder abgelassene Flüssigkeit eine veränderte Lage einer Körper- oder Gewebestruktur ergeben kann und möglicherweise der Drainageplan geändert werden muss, zum Beispiel durch die Neuplatzierung eines Katheters oder die Veränderung von Parametern.

Die Erfindung bezieht sich gemäß einem weiteren Aspekt auf ein Computerprogramm, welches in den Speicher eines Computers geladen werden kann und Softwarecodeabschnitte umfasst, mit welchen ein oder mehrere Schritte des oben beschriebenen Verfahrens ausgeführt werden können, wenn das Programm auf einem Computer läuft. Weiterhin bezieht sich die Erfindung auf ein Computerprogrammprodukt, das auf einem computergeeignetem Medium oder Datenträger gespeichert ist und das gerade beschriebene Computerprogramm umfasst.

Nach einem weiteren Aspekt bezieht sich die Erfindung auf eine Vorrichtung zur Simulation des Abflusses von Flüssigkeit aus einem Körper mit einer Datenerfassungsvorrichtung zur Erfassung von Strukturdaten des Körpers und/oder der Lage einer Flüssigkeit in einem Körper, wie zum Beispiel einem Kernspintomographen und einem Computersystem zur Bestimmung der Anordnung mindestens eines Katheters an einem Körper und/oder mit einer Eingabevorrichtung zur Eingabe der Position mindestens eines Katheters zur Simulation des Abflusses von Flüssigkeit aus dem Körper, wobei die durch die Datenerfassungsvorrichtung ermittelten körperspezifischen und flüssigkeitsbezogenen Daten verwendet werden.

Allgemein bezieht sich die vorliegende Erfindung auf eine Vorrichtung, mit welcher eine oder mehrere der oben beschriebenen Verfahrensschritte durchgeführt werden können. Beispielsweise sind hierzu Datenbanken zur Speicherung von Parametern oder Eigenschaften eines oder mehrerer Katheter, bestimmter Körperparameter, Flüssigkeitsparameter und/oder Drainageparameter vorhanden.

Zur Durchführung der Drainage ist vorteilhaft eine Vorrichtung zum Einstellen der Flussrate bei der Drainage vorgesehen, wie zum Beispiel eine Pumpe, welche in der Lage ist, eine gewünschte Saugwirkung oder einen Unterdruck zu erzeugen, um eine Drainage wie durch die erfindungsgemäßen Verfahren geplant und/oder simuliert durchführen zu können.

Zum Navigieren eines oder mehrerer Katheter können, um eine optimale Platzierung und einen optimalen Sitz dieser Katheter zu gewährleisten, bekannte Verfahren unter Verwendung von Markern verwendet werden, wie zum Beispiel das von der Anmelderin vertriebene VectorVision®-System.

Bezüglich einer in Verbindung mit der Erfindung verwendbaren Vorrichtung und Verfahren zur Verabreichung einer Substanz wird auf die am 30. November 2001 eingereichte europäische Patentanmeldung Nr. 01 128 614.3 der Anmelderin verwiesen.

Die Erfindung wird nachfolgend anhand von bevorzugten Ausführungsbeispielen beschrieben. Es zeigen:
- Figur 1: schematisch ein Schaubild eines Verfahrens zur Planung und Durchführung einer Drainage;
- Figur 2: ein vereinfachtes Ablaufdiagramm einer erfindungsgemäß durchgeführten Drainage; und
- Figur 3: eine Vorrichtung, welche bei der Planung und Durchführung einer Drainage verwendet werden kann.

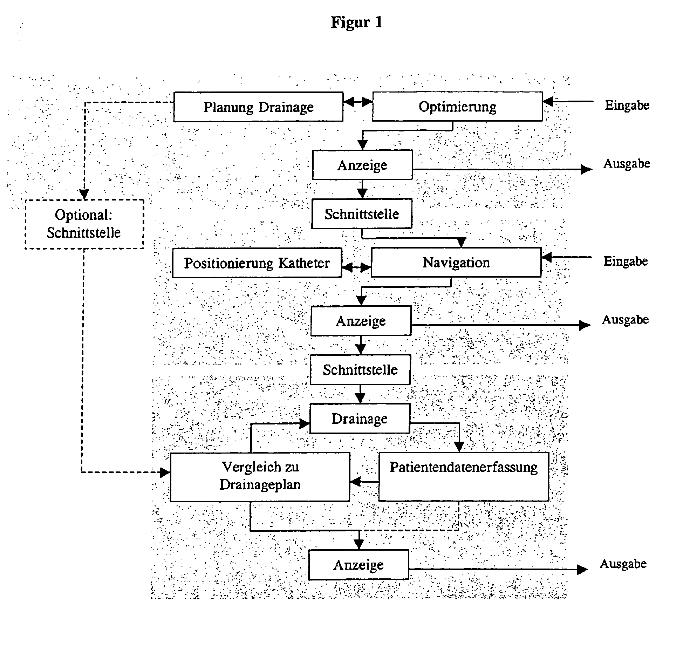

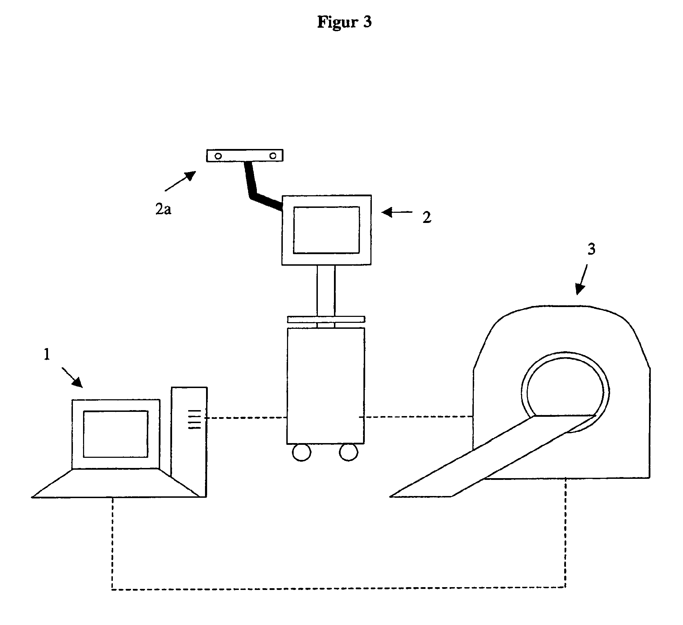

Figur 1 zeigt schematisch ein Ablaufdiagramm für die erfindungsgemäße Vorbereitung einer Drainage. Wie in Figur 1 gezeigt, werden Patientendaten zum Beispiel von einem Kernspintomographen eingegeben, welche verwendet werden, um einen oder mehrere bestimmte Bereiche zur Positionierung von Kathetern für die Drainage zu ermitteln und die durchzuführende Drainage zu planen. Diese Daten können zum Beispiel durch das Kernspinresonanz-System 3, wie in Figur 3 schematisch gezeigt, nach der Untersuchung eines zu behandelnden Patienten gewonnen werden. Unter Verwendung von zum Beispiel in Datenbanken abgespeicherten Parametern für die Eigenschaften der Gewebestrukturen und für verschiedene Kathetertypen kann nach der Ermittlung der genauen Lage der zu drainierenden interstitiellen Flüssigkeit und der Körper- oder Gewebestruktur ein oder mehrere für die Drainage geeignete Katheter ausgewählt werden. Die durch zum Beispiel das Kernspinresonanz-Verfahren gewonnenen Körper- oder Patientenparameter können zusammen mit den Katheterparametern und den zum Beispiel auch in Datenbanken gespeicherten Flüssigkeitsparametern verwendet werden, um die Drainage zu planen. Dabei kann der Verlauf der durchzuführenden Drainage unter der Randbedingung optimiert werden, dass ein möglichst großer Anteil der interstitiellen Flüssigkeit aus der Körperstruktur oder dem Zielgewebe entfernt wird, wobei möglichst wenige Eingriffe durchgeführt werden sollen. Allgemein sollten möglichst wenige Katheter oder Nadeln platziert werden, welche durch möglichst wenige Zugänge zugeführt werden. Diese optimierte Planung der Drainage wird über eine Anzeige ausgegeben, so dass zum Beispiel eine zweidimensionale oder dreidimensionale Darstellung durch Abbildung verschiedener Schnittebenen ausgegeben werden kann, um das Ergebnis der Drainageplanung anzuzeigen.

Der so erstellte Drainageplan wird über eine Schnittstelle an ein Navigationssystem übermittelt, wie beispielsweise das in Figur 3 schematisch gezeigte VectorVision®-System, um basierend auf den Planungsdaten den oder die ausgewählten Katheter an den vorgegebenen Stellen am Körper zu positionieren. Die Positionierung kann automatisch zum Beispiel unter Verwendung eines Roboters, oder durch das Navigationssystem geführt, von Hand erfolgen, wobei auf einer Anzeigevorrichtung dargestellt werden kann, ob ein Katheter richtig positioniert ist oder noch in eine bestimmte Richtung bewegt werden muss.

Nach erfolgter Positionierung des oder der Katheter(s) wird die eigentliche Drainage mit den durch die Planung vorgegebenen Drainageparametern, wie zum Beispiel einer sich zeitlich verändernden oder konstanten Flussrate durchgeführt. Dabei wird wiederum eine Patientendatenerfassung durchgeführt, um die tatsächliche Verteilung der Flüssigkeit im Körper oder Gewebe zu ermitteln. Unter Verwendung der durch die Planung vorgegebenen Parameter und der darauf basierenden Simulationsergebnisse für die Drainage wird ein Vergleich zwischen tatsächlicher Drainage, insbesondere Verteilung der teilweise drainierten Flüssigkeit und vorausbestimmter Flüssigkeitsverteilung durchgeführt und gegebenenfalls werden die Parameter, wie zum Beispiel die Flussrate, die Abflussmenge oder ein an dem Katheter anliegender Druck oder Sog zur Durchführung der Drainage bevorzugt unter Berücksichtigung von bekannten Wirkmechanismen abgeändert, um das gewünschte, geplante Drainageergebnis zu erhalten. Wiederum kann die gemessene tatsächliche Verteilung der interstitiellen Flüssigkeit bevorzugt zusammen mit eventuellen Abweichungen und Korrekturverfahren über eine Anzeige ausgegeben werden, um es zum Beispiel einer Bedienperson zu ermöglich manuell in das Drainageverfahren einzugreifen.

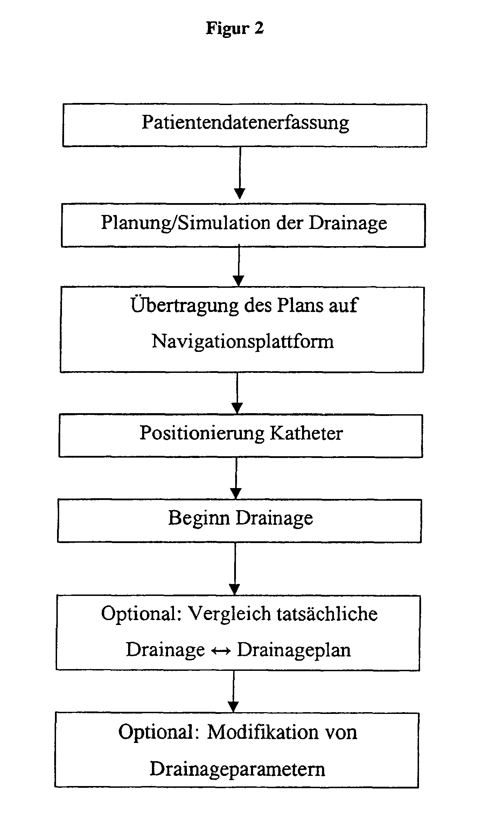

Figur 2 zeigt schematisch einen vereinfachten Ablauf einer erfindungsgemäßen Planung einer Drainage. Zunächst werden Patientendaten durch ein bildgebendes diagnostisches Verfahren wie beispielsweise ein Kernspinresonanz-Verfahren erfasst, um die aktuellen Patientenparameter wie zum Beispiel Gewebedichte, Druck und Lage einer zu drainierenden Flüssigkeit zu erhalten. Unter Verwendung der so ermittelten Patientenparameter sowie mit aus einer Datenbank erhaltenen und/oder für eine bestimmte Drainage vorgegebenen Katheter- und Drainageparametern wird die Drainage geplant und/oder simuliert. Basierend auf den so ermittelten Parameterdaten wird der Drainageplan an eine Navigationsplattform weitergegeben, mit welcher der oder die Katheter wie im Drainageplan vorgesehen am Patienten positioniert werden sollen. Die Drainage beginnt nach der Positionierung der Katheter und wird mit den geplanten und gegebenenfalls simulierten Parametern durchgeführt, wobei optional, wie in Figur 1 und 2 gezeigt, ein Vergleich der tatsächlich durchgeführten Drainage mit dem Drainageplan durchgeführt wird und bei Abweichungen eine Modifikation der entsprechenden Parameter bevorzugt unter Ausnutzung bekannter Wirkmechanismen vorgenommen wird.

Figur 3 zeigt schematisch eine Vorrichtung, welche zur Planung und Durchführung einer erfindungsgemäßen Drainage verwendet werden kann. Patientendaten werden in einem Kernspintomographen 3 erhalten und an ein Planungssystem 1 sowie ein Navigationssystem 2 weitergegeben. Mit dem Navigationssystem 2 werden unter Verwendung von zum Beispiel bekannten an einem oder mehreren Kathetern angebrachten Reflektoren oder Markern der oder die Katheter an einer gewünschten Stelle an einem Körper positioniert, wobei Positionsdaten der Marker durch IR-Kameras 2a erfasst werden. Das Planungssystem 1 ermittelt für eine vorgegebene durchzuführende Drainage unter Verwendung der vom Kernspinresonanzsystem 3 ermittelten Patientenparameter die geeigneten Katheterparameter und Drainageparameter, um die Drainage durchzuführen.

## Patentansprüche

1. Verfahren zur Vorbereitung einer Drainage einer Flüssigkeit aus einem Körper mit Hilfe eines Katheters, bei dem körperspezifisch eine anatomische und/oder Gewebestruktur erfasst wird, die Lage und/oder Menge der Flüssigkeit im Köper erfasst wird, die erfassten Informationen ausgewertet werden, und basierend darauf, sowie basierend auf Parametern zur Beschreibung von Eigenschaften des Körpers, insbesondere zur Beschreibung des Flussverhaltens einer Flüssigkeit in dem Körper, eine optimale Positionierung des Katheters ermittelt wird, wobei Parameter zur Beschreibung von Eigenschaften des Körpers, insbesondere zur Beschreibung des Flussverhaltens einer Flüssigkeit in dem Körper, zur Simulation des Flüssigkeitsabflusses verwendet werden.

2. Verfahren nach Anspruch 1, wobei die körperspezifischen anatomischen und/oder Gewebestruktur-Daten durch ein Kemspinresonanzverfahren (MRI), ein Computertomographieverfahren (CT), ein Röntgenverfahren, SPECT- oder ein Ultraschallverfahren erfasst werden.

3. Verfahren nach einem der vorhergehenden Ansprüche, wobei mindestens ein Katheter durch ein Navigationsverfahren an dem Körper positioniert wird.

4. Verfahren nach einem der vorhergehenden Ansprüche, wobei in einer Datenbank Parameter der aus dem Körper abzulassenden Flüssigkeit gespeichert sind und zur Positionierung eines Katheters und/oder zur Simulation des Flüssigkeitsabflusses verwendet werden.

5. Verfahren nach einem der vorhergehenden Ansprüche, wobei in einer Datenbank Parameter eines oder mehrer Katheter gespeichert sind und zur Positionierung eines Katheters und/oder zur Simulation des Flüssigkeitsabflusses verwendet werden.

6. Verfahren nach einem der vorhergehenden Ansprüche, wobei Daten zur spezifischen Anpassung oder Modifikation mindestens eines vorgegebenen Standardkatheters individuell angepasst ausgegeben werden.

7. Verfahren nach einem der vorhergehenden Ansprüche, wobei Drainageparameter, insbesondere eine Flussrate der abzulassenden Flüssigkeit und/oder ein an einen Katheter anzulegender Unterdruck ermittelt werden.

8. Verfahren nach einem der vorhergehenden Ansprüche, wobei nach einer durchgeführten Drainage eine weitere Datenerfassung zur Verifikation und/oder Modifikation des Verfahrens durchgeführt wird.

9. Computerprogramm, welches in den Speicher eines Computers geladen werden kann und Softwarecodeabschnitte umfasst, mit welchen die Schritte gemäß einem der vorhergehenden Ansprüche ausgeführt werden können, wenn das Programm auf einem Computer läuft.

10. Computerprogrammprodukt, das auf einem Computer geeignetem Medium oder Datenträger gespeichert ist und das Computerprogramm nach dem vorhergehenden Anspruch umfasst.

11. Vorrichtung zur Positionierung mindestens eines Katheters an einem Körper mit einer Datenerfassungsvorrichtung (3) zur Erfassung von Strukturdaten des Körpers und/oder der Lage einer Flüssigkeit in einem Körper, einem Datenträger zur Speicherung von Parametern zur Beschreibung von Eigenschaften des Körpers, insbesondere zur Beschreibung des Flussverhaltens einer Flüssigkeit in dem Körper und einem Computersystem (1) zur Auswertung der von dem Datenerfassungssystem (3) erfassten Daten zur Bestimmung der Position mindestens eines Katheters an einem Objekt zur Durchführung einer Drainage und zur Simulation des Flüssigkeitsabflusses.

12. Vorrichtung nach Anspruche 11 mit einer Datenbank zur Speicherung von Katheter-Parametern, Körperparametern, Flüssigkeitsparametern und/oder Drainageparametem.

13. Vorrichtung nach einem der Ansprüche 11 oder 12, mit einer Vorrichtung zur Einstellen der Flussrate der abzulassenden Flüssigkeit.

14. Vorrichtung nach einem der Ansprüche 11 bis 13, mit einem Navigationssystem zum Anbringen mindestens eines Katheters an einer bestimmten Stelle des Körpers.

## Claims

1. A method for preparing a drainage of a fluid from a body with the aid of a catheter, wherein an anatomical and/or tissue structure is detected body-specifically, the position and/or amount of the fluid in the body is detected, the captured information is evaluated, and an optimum position of the catheter is ascertained on the basis of this and on the basis of parameters for describing properties of the body, in particular for describing the flow characteristics of a fluid in the body, wherein parameters for describing properties of the body, in particular for describing the flow characteristics of a fluid in the body, are used to simulate the fluid drainage.

2. The method according to claim 1, wherein the body-specific anatomical and/or tissue structure data are captured using a nuclear spin resonance method (MRI), a computed tomography method (CT), an x-ray method, a SPECT method or an ultrasound method.

3. The method according to any one of the preceding claims, wherein at least one catheter is positioned on the body using a navigation method.

4. The method according to any one of the preceding claims, wherein parameters of the fluid to be drained from the body are stored in a database and used to position a catheter and/or to simulate the fluid drainage.

5. The method according to any one of the preceding claims, wherein parameters of one or more catheters are stored in a database and used to position a catheter and/or to simulate the fluid drainage.

6. The method according to any one of the preceding claims, wherein data are outputted, individually adapted, for specifically adapting or modifying at least one predetermined standard catheter.

7. The method according to any one of the preceding claims, wherein drainage parameters, in particular a flow rate of the fluid to be drained and/or a negative pressure to be applied to a catheter, are ascertained.

8. The method according to any one of the preceding claims, wherein data are additionally captured after a drainage has been performed, in order to verify and/or modify the method.

9. A computer program which can be loaded into the memory of a computer and which includes sections of software code using which the steps in accordance with any one of the preceding claims can be performed when the program is running on a computer.

10. A computer program product which is stored on a computer-compatible medium or data carrier and comprises the computer program according to the preceding claim.

11. A device for positioning at least one catheter on a body, comprising: a data capture device (3) for capturing structural data of the body and/or capturing the position of a fluid in a body; a data carrier for storing parameters for describing properties of the body, in particular for describing the flow characteristics of a fluid in the body; and a computer system (1) for evaluating the data captured by the data capture system (3) in order to determine the position of at least one catheter on an object in order to perform a drainage and to simulate the fluid drainage.

12. The device according to claim 11, comprising a database for storing catheter parameters, body parameters, fluid parameters and/or drainage parameters.

13. The device according to any one of claims 11 or 12, comprising a device for setting the flow rate of the fluid to be drained.

14. The device according to any one of claims 11 to 13, comprising a navigation system for arranging at least one catheter at a specific location on the body.

## Revendications

1. Procédé pour préparer l'évacuation par drainage d'un fluide présent dans un corps à l'aide d'un cathéter, comportant les étapes consistant à détecter une structure de tissu et/ou une structure anatomique de manière spécifique au corps, détecter la position et/ou la quantité du fluide dans le corps, évaluer les informations détectées et, sur la base de celles-ci ainsi que sur la base de paramètres pour décrire des caractéristiques du corps, en particulier pour décrire le comportement à l'écoulement d'un fluide dans le corps, déterminer un positionnement optimal du cathéter, les paramètres pour décrire des caractéristiques du corps, en particulier pour décrire le comportement à l'écoulement d'un fluide dans le corps, étant utilisés pour simuler l'évacuation de fluide.

2. Procédé selon la revendication 1, comportant l'étape consistant à acquérir les données anatomiques et/ou les données de structure de tissu spécifiques au corps par un procédé de résonance magnétique nucléaire (MRI), un procédé de tomographie numérique (CT), un procédé aux rayons X, un procédé SPECT ou un procédé aux ultrasons.

3. Procédé selon l'une quelconque des revendications précédentes, comportant l'étape consistant à positionner au moins un cathéter sur le corps en utilisant un procédé de navigation.

4. Procédé selon l'une quelconque des revendications précédentes, comportant les étapes consistant à mémoriser des paramètres du fluide à évacuer du corps dans une base de données et les utiliser pour positionner un cathéter et/ou pour simuler l'évacuation de fluide.

5. Procédé selon l'une quelconque des revendications précédentes, comportant les étapes consistant à mémoriser des paramètres d'un ou plusieurs cathéters dans une base de données et les utiliser pour positionner un cathéter et/ou pour simuler l'évacuation de fluide.

6. Procédé selon l'une quelconque des revendications précédentes, comportant l'étape consistant à générer des données individuellement adaptées pour adapter ou modifier spécifiquement au moins un cathéter standard prédéterminé.

7. Procédé selon l'une quelconque des revendications précédentes, comportant l'étape consistant à déterminer des paramètres de drainage, en particulier un débit du fluide à évacuer et/ou une pression négative à appliquer à un cathéter.

8. Procédé selon l'une quelconque des revendications précédentes, comportant l'étape consistant à, après l'exécution d'un drainage, acquérir des données supplémentaires pour vérifier et/ou modifier le procédé.

9. Programme informatique pouvant être chargé dans la mémoire d'un ordinateur et comportant une partie de code logiciel, au moyen duquel les étapes selon l'une quelconque des revendications précédentes peuvent être exécutées lorsque le programme s'exécute sur un ordinateur.

10. Produit de programme informatique mémorisé sur un ordinateur, un support adapté ou un support de données, et comportant le programme informatique selon la revendication précédente.

11. Dispositif pour positionner au moins un cathéter sur un corps, comportant un dispositif d'acquisition de données (3) pour acquérir des données de structure du corps et/ou la position d'un fluide dans un corps, un support de données pour mémoriser des paramètres pour décrire des caractéristiques du corps, en particulier pour décrire le comportement à l'écoulement d'un fluide dans le corps, et un système informatique (1) pour évaluer les données acquises par le système d'acquisition de données (3) afin de déterminer la position d'au moins un cathéter sur un objet en exécutant un drainage ou de simuler l'évacuation de fluide.

12. Dispositif selon la revendication 11 comportant une base de données pour mémoriser des paramètres de cathéter, des paramètres corporels, des paramètres de fluide et/ou des paramètres de drainage.

13. Dispositif selon l'une quelconque des revendications 11 ou 12, comportant un dispositif pour régler le débit du fluide à évacuer.

14. Dispositif selon l'une quelconque des revendications 11 à 13, comportant un système de navigation pour fixer au moins un cathéter à un emplacement déterminé du corps.
